(19) Europäisches Patentamt
European Patent Office
Office européen des brevets

(11) Publication number: **0 009 722**
**B1**

(12) # EUROPEAN PATENT SPECIFICATION

(45) Date of publication of patent specification: **02.03.83**

(21) Application number: **79103536.3**

(22) Date of filing: **19.09.79**

(51) Int. Cl.³: **C 07 C 101/08,**
C 07 C 59/56,
C 07 C 59/48,
C 07 C 59/64,
C 07 C 79/46, C 07 B 19/00

(54) **Optically active complex, alanine.Ring-substituted mandelic acid, and the method for producing the same.**

(30) Priority: **22.09.78 JP 115988/78**

(43) Date of publication of application:
**16.04.80 Bulletin 80/8**

(45) Publication of the grant of the patent:
**02.03.83 Bulletin 83/9**

(84) Designated Contracting States:
**DE FR GB IT NL**

(56) References cited:
**DE - A - 2 812 041**

**CHEMICAL ABSTRACTS, vol. 77, no. 3, 17th July 1972, pages 541, no. 19976j**
**K. JOICHI et al.: "Asymmetric interaction between mandelic acid and aromatic alpha-amino acids in aqueous system"**

(73) Proprietor: **NIPPON KAYAKU KABUSHIKI KAISHA**
**2-1, Marunouchi-1-chome Chiyoda-ku**
**Tokyo 100 (JP)**

(72) Inventor: **Tashiro, Yasuhisa**
**No. 2-70-1, Nakadoori Tsurumi-ku**
**Yokohama-shi Kanagawa-ken (JP)**
Inventor: **Nagashima, Takashi**
**No. 1249-60, Ooaza Ooma**
**Kounosu-shi Saitama-ken (JP)**
Inventor: **Aoki, Shigeru**
**No. 3-22-3, Kamiikebukuro**
**Toshima-ku Tokyo (JP)**
Inventor: **Aboshi, Yuzo**
**No. 1-21-14, Wakamiya**
**Nakano-ku Tokyo (JP)**

(74) Representative: **Deufel, Paul, Dr. et al,**
**Patentanwälte Müller-Boré, Deufel Schön, Hertel**
**Siebertstrasse 4 Postfach 860720**
**D-8000 München 86 (DE)**

EP 0 009 722 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Optically active complex, alanine.ring-substituted mandelic acid, and the method for producing the same

The present invention relates to an optically active complex, alanine.ring-substituted mandelic acid, represented by the general formula:

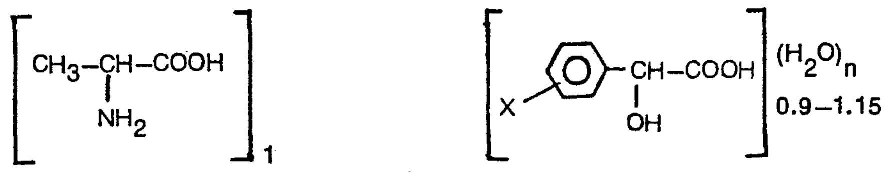

wherein X is p-chloro, p-methoxy, p-methyl or m-nitro, and n is 0 or 1/2 and a method for producing such complexes.

The novel substances of the present invention are useful as intermediates for optically active alanine or optically active ring-substituted mandelic acid.

Hitherto, for the optical resolution of alanine a method has been used in which alanine is converted to its N-benzoyl derivative and the derivative is in turn converted to a slightly soluble salt by reaction with an expensive alkaloid such as brucine or strychnine, and after removing the alkaloid which is released by decomposition of the salt, the thus obtained N-benzoyl derivative is hydrolyzed to obtain the optically active alanine. However, the above-mentioned method has unavoidable technical drawbacks.

Another method is known in which acylated alanine is optically resolved by amino-acylase, however, since in this method it is necessary to acylate alanine to obtain an acyl-derivative, this method is not advantageous.

On the other hand, as a method of optically resolving ring-substituted mandelic acid only one method is known, in which ephedrine is reacted with p-chloromandelic acid and the reaction product is resolved, there are no other known methods for optically resolving other ring-substituted mandelic acids.

The inventors previously discovered a method of optical resolution using a complex of mandelic acid with alanine (German Offen. 2812041). It was previously known (vide chem.Abst. Vol 77, No. 3 19976(j)) that certain aromatic acids formed complexes with mandelic acid, but since such complex formation was thought to result from the interaction of the two aromatic rings, it was not to be expected that mandelic acid, or indeed substituted mandelic acids would form complexes with nonaromatic amino acids, such as alanine.

During the study of the combination of ring-substituted mandelic acids and alanine the inventors have now found that a ring-substituted mandelic acid, such as that mentioned above, forms a complex with alanine and that owing to the difference of solubility between two complexes, when an optically active isomer of one of the pair comprising alanine and ring-substituted mandelic acid is used, the racemic compound of the other of the above-mentioned pair may be resolved optically. For instance, by using L-alanine an optically active ring-substituted mandelic acid is obtained and conversely, by using optically active ring-substituted mandelic acid an optically active alanine is obtained.

The present invention is based on the above-mentioned findings.

In order to produce the desired substance of the present invention an optically active ring-substituted mandelic acid is reacted in a solvent with DL-alanine, or optically active alanine is reacted in a solvent with the racemic compound, DL- ring-substituted mandelic acid, to form a complex. The slightly soluble complex may then be selectively crystallized out by cooling the solution, by condensing the solution, if necessary, or by adding another solvent which reduces the solubility of the complex to effect the separation of the solid and the liquid. It is not necessary to add a so called "seed" to cause the crystallisation to proceed but a small amount of the slightly soluble complex may be added in order to accelerate the crystallisation.

The substance to be optically resolved may be a racemic mixture of L-isomer and D-isomer or a mixture in which one isomer is present in more than half the molar amount of the mixture against its antipode.

The solvent used for the purpose of the invention is not especially limited, provided the slightly soluble complex and the easily soluble complex dissolves in the solvent at a temperature of between room temperature and its boiling point, and the slightly soluble complex separates out by cooling or concentrating the solution or by adding another solvent to the solution. However, water, a hydrophilic organic solvent, for instance, methanol, ethanol, acetone, etc. or an aqueous solution thereof is preferable.

The reaction is carried out at a temperature of higher than 0°C and lower than the boiling point of the solvent used in the reaction, however, it is preferable to carry out the reaction at a temperature between 50°C and the boiling point of the solvent used. The temperature at which the complex

separates out is not especially limited provided it is lower than the boiling point of the solvent used. However, it is preferably a temperature of between 0°C and 50°C.

The pH of the reaction system is preferably in the range of 1.0—4.0.

The mole ratio of alanine to the ring-substituted mandelic acid is 1.0 : 0.3—4.0, preferably 1.0 : 1.0 ; 3.0. The concentration of alanine in the solvent is preferably from 10 to 70% by weight.

When the optically active complex obtained is not optically pure, it is easily recrystallised to afford the optically pure complex.

The composition of the complex of the present invention is:

$(\text{alanine})_1\text{-(ring-substituted mandelic acid)}_{0.9-1.15}$.

The actual composition, melting point, specific rotary power and elementary analytical data are shown in Table 1.

TABLE 1

| Complex compound | Specific rotatory power $[\alpha]^{25}_{D}$ C = 1, $H_2O$ | Composition MA (mol) / ALa (mol) | Elementary analysis | | | | | | Melting point °C |
|---|---|---|---|---|---|---|---|---|---|
| | | | C % | | H % | | N % | | |
| | | | Calcd. | Found | Calcd. | Found | Calcd. | Found | |
| D-ALa·L-p-ClMA | +81.16° | 0.962 | 47.83 | 47.67 | 5.15 | 5.01 | 5.22 | 5.09 | Do not show a clear melting point |
| L-ALa·D-p-ClMA | −83.17° | 0.961 | 47.82 | 47.13 | 5.16 | 5.32 | 5.22 | 5.06 | |
| D-ALa·D-p-ClMA | −89.42° | 1.050 | 47.99 | 47.98 | 5.09 | 5.09 | 4.98 | 4.88 | |
| L-ALa·L-p-ClMA | +89.42° | 1.031 | 48.04 | 47.66 | 5.08 | 5.05 | 4.91 | 4.88 | |
| D-ALa·D-p-MeOMA | −98.88° | 1.133 | 53.64 | 53.66 | 6.25 | 6.29 | 4.74 | 4.46 | |
| L-ALa·L-p-MeOMA | +94.70° | 1.007 | 53.16 | 52.59 | 6.31 | 6.31 | 5.14 | 5.05 | |
| D-ALa·L-p-MeOMA | +87.91° | 0.959 | 52.95 | 52.26 | 6.34 | 6.26 | 5.31 | 5.35 | |
| L-ALa·D-p-MeOMA | −88.90° | 0.947 | 52.90 | 52.44 | 6.35 | 6.33 | 5.35 | 5.32 | |
| D-ALa·D-m-$NO_2$MA | −78.32° | 0.981 | 46.12 | 45.64 | 4.95 | 4.88 | 9.82 | 9.72 | |
| L-ALa·L-m-$NO_2$MA | +77.13° | 0.981 | 46.12 | 45.91 | 4.95 | 4.88 | 9.82 | 9.50 | |
| D-ALa·L-m-$NO_2$MA | +72.56° | 1.000 | —* | — | — | — | — | — | |
| L-ALa·D-m-$NO_2$MA | −71.75° | 1.000 | —* | — | — | — | — | — | |
| D-ALa·D-p-MeMA | −94.23° | 1.026 | 56.60 | 55.13 | 6.70 | 5.31 | 5.40 | 5.18 | |
| L-ALa·L-p-MeMA | +95.31° | 1.032 | 56.54 | 55.21 | 6.68 | 5.39 | 5.53 | 5.22 | |
| D-ALa·L-p-MeMA 1/2 $H_2O$ | +85.40 | 0.929 | 54.43 | 53.49 | 6.87 | 6.72 | 5.58 | 5.32 | |
| L-ALa·D-p-MeMA 1/2 $H_2O$ | −86.63° | 0.929 | 54.43 | 54.18 | 6.87 | 6.67 | 5.58 | 5.43 | |

Notes:
ALa : Alanine
p-ClMA : p-Chloromandelic acid
p-MeOMA : p-Methoxymandelic acid
m-$NO_2$MA : m-Nitromandelic acid
p-MeMA : p-Methylmandelic acid

* Not analyzed because it was an oil.

The optically active alanine is obtainable by dissolving the complex obtained by the present invention in water, a hydrophilic organic solvent or its aqueous solution, decomposing the complex by adding a mineral acid, separating the ring-substituted mandelic acid with the addition of an organic solvent such as ether, etc. or separating alanine from the ring-substituted mandelic acid using a strongly acidic ion-exchange resin.

D-alanine obtained by decomposing the substance of the present invention is important as a raw material in the production of antibiotics. L-alanine is important as a component of solutions for amino acid infusion and as a food additive. The optically active ring-substituted mandelic acid is useful as an optical resolution agent and its D-isomer is useful as a raw material for antibiotics of the beta-lactam type.

Optically active alanine or an optically active ring-substituted mandelic acid may be repeatedly utilised as the optical resolution agent.

As has been described, the desired substance of the present invention is useful as an intermediate in cases where an optically active compound is prepared from DL-alanine or a DL-ring-substituted mandelic acid, and the method of optical resolution via the intermediate has the following advantages compared to the usual methods:

1. It is not necessary to convert the alanine to be resolved into a derivative, and
2. Water may be used as the solvent in the process.

The following are exemplifications of the present invention.

Example 1

DL-p-chloromandelic acid (16.43 g = 0.008 M) and L-alanine (5.23 g = 0.059 M) were mixed in 26.4 ml of water, and the mixture was heated to 70°C to form a solution. After cooling the solution to room temperature and then stirring the cooled solution for one hour at room temperature, the separated crystals were collected by filtration, washed with water and dried. The thus obtained crystals, weighing 7.89 g, were a raw complex, D-p-chloromandelic acid-L-alanine, showing a specific rotatory power $[\alpha]_D^{25} = -50.46°$ (c = 1, solvent: Water).

After mixing 7.5 g of the raw complex with 15 ml of water and heating the mixture to form a solution, it was cooled to room temperature and then stirred for one hour. The crystals separated out from the solution. After collecting the crystals by filtration and washing with water, the crystals were dried to obtain 4.36 g of an optically pure complex D-p-chloromandelic acid-L-alanine, with a specific rotary power of $[\alpha]_D^{25} = -83.17°$ (c = 1, water).

Examples 2—4

The following ring-substituted mandelic acids were optically resolved by the same procedure as in Example 1 using optically active alanine. The results are shown in Table 2.

TABLE 2

| Example No. | Alanine g | Ring-substituted DL-mandelic acid g | | Raw crystal Yield g | Raw crystal $[\alpha]_D^{25}$ (c=1 $H_2O$) | Purified crystal Yield g | Purified crystal $[\alpha]_D^{25}$ (c=1 $H_2O$) | Composition of complex |
|---|---|---|---|---|---|---|---|---|
| 2 | D-Ala 6.60 | p-methoxy-mandelic acid | 19.79 | 5.44 | −88.85° | 4.08 | −98.88° | D-p-MeOMA· D-Ala |
| 3 | L-Ala 7.50 | m-nitro-mandelic acid | 25.00 | 9.87 | +69.7° | 7.20 | +77.1° | L-m-$NO_2$MA· L-Ala |
| 4 | D-Ala 16.19 | p-methyl-mandelic acid | 11.19 | 8.40 | +84.13° | 7.58 | +85.40° | L-p-MeMA· D-Ala 1/2$H_2O$ |

Note: Amount of water used in Examples 2 – 4 was 33.3, 20.8 and 10.1 ml, respectively.

Example 5

DL-alanine (5.23 g) and D-p-chloromandelic acid (16.4 g) were mixed with 27 ml of water, and the mixture was heated to 70°C to dissolve the solids. After leaving the solution at room temperature, the

crystals which separated out were collected by filtration and washed with 2 ml of water. By drying the washed crystals, 9.08 g of a complex of L-alanine·D-p-chloromandelic acid with a specific rotatory power of $[\alpha]_D^{25} = -46.3°$ (c = 1, water) were obtained. On recrystallization of 8.5 g of the crystals from 18 ml of water, purified crystals weighing 3.79 g were obtained. Its specific rotatory power was $[\alpha]_D^{25} = -83.1°$ (c = 1, water).

Examples 6 and 7

The results of optical resolution of DL-alanine using each of the optically active ring-substituted mandelic acids as in Example 5 are shown in table 3.

TABLE 3

| Example No. | Optically active mandelic acid | | DL-Alanine g | Crude crystal | | Purified crystal | | Composition of complex |
|---|---|---|---|---|---|---|---|---|
| | | | | Yield g | $[\alpha]_D^{25}$ (c=1 $H_2O$) | Yield g | $[\alpha]_D^{25}$ (c=1 $H_2O$) | |
| 6 | D-m-nitro-mandelic acid | 12.5g | 7.5 | 8.40 | +70.2° | 7.8 | +76.2° | D-m-$NO_2$MA·D-Ala |
| 7 | D-p-methoxy-mandelic acid | 19.8g | 6.7 | 6.05 | +76.5° | 4.3 | +94.3° | L-p-MeOMA·L-Ala |

Note: Amount of water used in Examples 6 and 7 was 20.8 and 34 ml, respectively.

Example 8

DL-Alanine (7.5 g) and L-m-nitromandelic acid (12.5 g) were added to 35 ml of 33.3% aqueous methanolic solution and after dissolving the solids by heating the mixture, the solution was stirred for 2 hours at room temperature. Then the separated crystals from the cooled solution were collected by filtration and washed with 3 ml of water. On drying the crystals, 9.56 g of a complex, L-alanine·L-m-nitromandelic acid, with a specific rotatory power of $[\alpha]_D^{25} = -63.2°$ (c = 1, water) was obtained.

On recrystallization of the raw complex, 5.6 g of purified complex, L-alanine·L-m-nitromandelic acid was obtained.

Referential Example 1

A complex, D-p-chloromandelic acid·L-alanine (5.0 g) obtained in Example 1 was dissolved in 30 ml of water and the solution was infused into 30 ml of a strongly acidic ion-exchange resin, Dowex 50W ($H^+$) ("Dowex" is a trademark of Dow Chemical Co., U.S.A.). On concentrating and drying, 1.87 g of D-p-chloromandelic acid was obtained. Its specific rotatory power was $[\alpha]_D^{25} = -133.96°$ (c = 1, water.

Referential Example 2

A complex, D-m-nitromandelic acid·D-alanine (5.0 g) obtained in Example 6 was dissolved in 30 ml of water and the solution was infused into 20 ml of a weakly basic ion-exchange resin Diaion WA—20 ($OH^-$) ("Diaion" is a trademark of Mitsubishi Chemical Industries Ltd.). On concentrating the effluent to dryness, 1.52 g of D-alanine was obtained. Its specific rotatory power was $[\alpha]_D^{25} = -14.7°$ (c = 1, 6N—HCl).

## Claims

1. An optically active complex, alanine-ring-substituted mandelic acid, represented by the general formula:

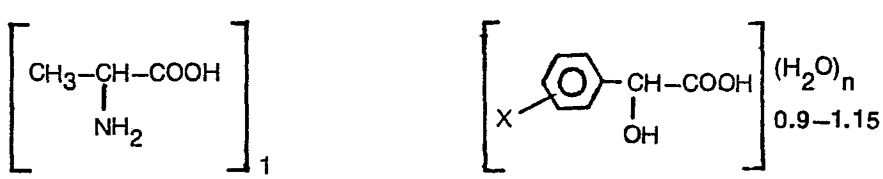

wherein X is p-chloro, p-methoxy, p-methyl or m-nitro, and n is 0 or 1/2.

2. D-alanine-L-p-chlormandelic acid.
3. D-alanine.D-p-chlormandelic acid.
4. L-alanine.L-p-chlormandelic acid.
5. L-alanine.D-p-chlormandelic acid.
6. D-alanine.L-p-methoxymandelic acid.
7. D-alanine.D-p-methoxymandelic acid.
8. L-alanine.L-p-methoxymandelic acid.
9. L-alanine.D-p-methoxymandelic acid.
10. D-alanine.L-m-nitromandelic acid.
11. D-alanine.D-m-nitromandelic acid.
12. L-alanine.L-m-nitromandelic acid.
13. L-alanine.D-m-nitromandelic acid.
14. D-alanine.L-p-methylmandelic acid.1/2 $H_2O$
15. D-alanine.D-p-methylmandelic acid.
16. L-alanine.L-p-methylmandelic acid.
17. L-alanine.D-p-methylmandelic acid.1/2 $H_2O$

18. A method for producing an optically active complex, alanine-ring-substituted mandelic acid according to Claim 1, characterised in that an optically active isomer of ring-substituted mandelic acid represented by the general formula

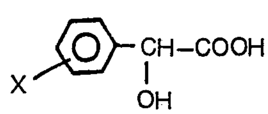

wherein X is p-chloro, p-methoxy, p-methyl or m-nitro, is reacted with DL-alanine in a solvent, or optically active alanine is reacted with DL-ring-substituted mandelic acid, as defined above in a solvent at a temperature of greater than 0°C and below the boiling point of the solvent the molar ratio of alanine to the mandelic acid derivative being 1.0 : 0.3—4.0.

**Revendications**

1. Complexe alanine-acide mandélique substitué sur le noyau, optiquement actif, répondant à la formule générale

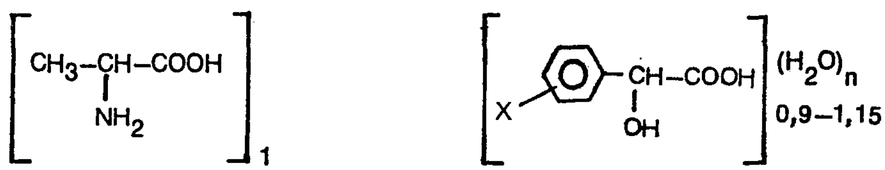

dans laquelle X représente un substituant p-chloro, p-méthoxy, p-méthyle ou m-nitro, et n est égal à 0 ou à 1/2.

2. Complexe D-alanine.acide L-p-chloromandélique.
3. Complexe D-alanine.acide D-p-chloromandélique.
4. Complexe L-alanine.acide L-p-chloromandélique.
5. Complexe L-alanine.acide D-p-chloromandélique.
6. Complexe D-alanine.acide L-p-méthoxymandélique.
7. Complexe D-alanine.acide D-p-méthoxymandélique.
8. Complexe L-alanine.acide L-p-méthoxymandélique.
9. Complexe L-alanine.acide D-p-méthoxymandélique.
10. Complexe D-alanine.acide L-m-nitromandélique.
11. Complexe D-alanine.acide D-m-nitromandélique.
12. Complexe L-alanine.acide L-m-nitromandélique.
13. Complexe L-alanine.acide D-m-nitromandélique.
14. Complexe D-alanine.acide L-p-méthylmandélique.1/2 $H_2O$.
15. Complexe D-alanine.acide D-p-méthylmandélique.
16. Complexe L-alanine.acide L-p-méthylmandélique.
17. Complexe L-alanine.acide D-p-méthylmandélique.1/2 $H_2O$.

18. Procédé de préparation d'un complexe alanine-acide mandélique substitué sur le noyau, optiquement actif selon la revendication 1, caractérisé en ce que l'on fait réagir un acide mandélique substitué sur le noyau optiquement actif et répondant à la formule générale

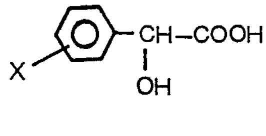

dans laquelle X représente un substituant p-chloro, p-méthoxy, p-méthyle ou m-nitro, avec la DL-alanine dans un solvant, ou bien on fait réagir de l'alanine optiquement active avec un acide DL-mandélique substitué sur le noyau tel que défini ci-dessus dans un solvant à une température supérieure à 0°C et inférieure au point d'ébullition du solvant, à un rapport molaire alanine/acide mandélique substitué sur le noyau de 1,0 : 0,3 à 4,0.

**Patentansprüche**

1. Optisch aktiver Komplex aus Alanin-Ring-substituierter Mandelsäure der allgemeinen Formel

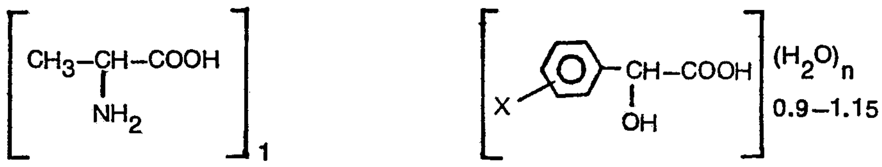

worin X für p-Chlor, p-Methoxy, p-Methyl oder m-Nitro steht und n 0 oder 1/2 ist.

2. D-Alanin-L-p-chlormandelsäure.
3. D-Alanin-D-p-chlormandelsäure.
4. L-Alanin-L-p-chlormandelsäure.
5. L-Alanin-D-p-chlormandelsäure.
6. D-Alanin-L-p-methoxymandelsäure.
7. D-Alanin-D-p-methoxymandelsäure.
8. L-Alanin-L-p-methoxymandelsäure.
9. L-Alanin-D-p-methoxymandelsäure.
10. D-Alanin-L-m-nitromandelsäure.
11. D-Alanin-D-m-nitromandelsäure.
12. L-Alanin-L-m-nitromandelsäure.
13. L-Alanin-D-m-nitromandelsäure.
14. D-Alanin-L-p-methylmandelsäure-1/2 $H_2O$.
15. D-Alanin-D-p-methylmandelsäure.
16. L-Alanin-L-p-methylmandelsäure.
17. L-Alanin-D-p-methylmandelsäure-1/2 $H_2O$.
18. Verfahren zur Herstellung eines optisch aktiven Komplexes, und zwar Alanin-Ring-substituierter Mandelsäure gemäß Anspruch 1, dadurch gekennzeichnet, daß ein optisch aktives Isomeres einer ringsubstituierten Mandelsäure der allgemeinen Formel

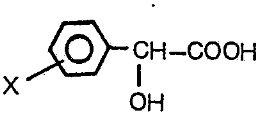

worin X für p-Chlor, p-Methoxy, p-Methyl oder m-Nitro steht, mit DL-Alanin in einem Lösungsmittel oder ein optisch aktives Alanin mit DL-Ring-substituierter Mandelsäure, wie vorstehend definiert, in einem Lösungsmittel bei einer Temperatur von mehr als 0°C und unterhalb des Siedepunkt des Lösungsmittel zur Umsetzung gebracht wird, wobei das Molverhältnis von Alanin zu dem Mandelsäurederivat 1,0.0,3—4,0 beträgt.